## Europäisches Patentamt

## European Patent Office

## Office européen des brevets

(19)

(11) Veröffentlichungsnummer: **0 072 920**
**B1**

(12) **EUROPÄISCHE PATENTSCHRIFT**

(45) Veröffentlichungstag der Patentschrift:
**12.12.84**

(51) Int. Cl.³: **C 07 C  1/20,** C 07 C  11/02,
B 01 J  29/38, B 01 J  37/26

(21) Anmeldenummer: **82106393.0**

(22) Anmeldetag: **16.07.82**

(54) **Verfahren zur Herstellung von Olefinen aus Methanol und/oder Dimethylether.**

(30) Priorität: **13.08.81  DE 3132024**

(43) Veröffentlichungstag der Anmeldung:
**02.03.83 Patentblatt 83/9**

(45) Bekanntmachung des Hinweises auf die Patenterteilung:
**12.12.84 Patentblatt 84/50**

(84) Benannte Vertragsstaaten:
**BE DE FR GB NL**

(56) Entgegenhaltungen:
**GB - A - 1 386 801**
**GB - A - 1 386 802**
**US - A - 4 297 335**

(73) Patentinhaber: **BASF Aktiengesellschaft,
Carl-Bosch-Strasse 38, D-6700 Ludwigshafen (DE)**

(72) Erfinder: **Hoelderich, Wolfgang, Dr., Mannheimer
Strasse 18C, D-6710 Frankenthal (DE)**
Erfinder: **Mross, Wolf Dieter, Dr.,
Anselm-Feuerbach-Strasse 21, D-6710 Frankenthal (DE)**
Erfinder: **Schwarzmann, Matthias, Dr.,
Carl-Bosch-Strasse 54, D-6703 Limburgerhof (DE)**

## Beschreibung

In den vergangenen Jahren sind Verfahren entwickelt worden, die die Herstellung von Olefinen aus Methanol und/oder Dimethylether zum Gegenstand haben. Ein solches Verfahren ist beispielsweise in der DE-OS 2 615 150 beschrieben. Als Katalysator wird ein Aluminosilikatzeolith vom Typ ZSM-5 verwendet, der eigentlich ein Aromatisierungskatalysator ist. Die Umsetzung wird im bekannten Falle durch verschiedene Maßnahmen, insbesondere durch die Verkürzung der Verweilzeit, in Richtung Olefinbildung gelenkt. Weitere die Olefinbildung begünstigende Parameter sind insbesondere die Verdünnung von Methanol bzw. Dimethylether mit Inertgasen bzw. Wasserdampf oder die Verdünnung des Katalysators mit Bindemitteln. Die Erfahrung zeigt, daß hohe Olefinausbeuten nur durch eine sehr starke Verdünnung von Methanol und/oder Dimethylether mit Inertgas oder Wasserdampf zu erzielen sind. Andere bekannte Verfahren haben als Nachteil eine geringe Belastbarkeit und eine schnelle Verkohlung des Katalysators. Es besteht daher Interesse an einem Verfahren, das die vollständige Umsetzung von Methanol und/oder Dimethylether in ein überwiegend aus $C_2-C_4$-Olefinen bestehendes Kohlenwasserstoffgemisch bei hoher Laufzeit des Katalysators ermöglicht.

Aus GB-PS 1 386 801 ist auch ein Verfahren bekannt, bei dem man aluminiumsilikathaltige Katalysatoren mit Fluorwasserstoff behandelt hat. Bei dieser von der vorliegenden Reaktion verschiedenen Anwendung werden nichtzeolithische Aluminosilikate behandelt, um den Aluminiumgehalt herabzusetzen, während im Falle der Erfindung die Aufgabe gestellt war, den Aluminium- bzw. Borgehalt zu erhalten und durch die HF-Behandlung die Lewis-sauren Zentren der Zeolithe zu blockieren und die Brönstedt-Acidität zu erhöhen.

Es wurde nun gefunden, daß man Olefine in hoher Ausbeute durch Umsetzung von Methanol und/oder Dimethylether bei erhöhter Temperatur in Gegenwart von Zeolith-Katalysatoren erhält, wenn man mit Fluorwasserstoff behandelte Zeolithkatalysatoren vom Pentasiltyp verwendet.

Die Zeolithkatalysatoren können für sich und auch mit Bindemitteln vermischt angewendet werden. Als Bindemittel kann man Kieselsäuren, Magnesiumoxid, Aluminiumphosphat verwenden. Vorteilhaft wendet man Aluminiumoxide an. Die Katalysatoren können unvermischt z. B. zu Tabletten verpreßt werden oder mit Bindemitteln vermischt und in üblicher Form verstrangt werden. Man kann den Katalysatoren 10 bis 90 Gew.-% Bindemittel beimischen.

Die Behandlung mit Fluorwasserstoff wird vorzugsweise direkt an dem Zeolithkatalysator vor der Vermischung und Verarbeitung in der Strangpresse vorgenommen. Man kann aber auch den fertigen verstrangten Katalysator der HF-Behandlung unterwerfen.

Eine besondere Ausführungsform des Verfahrens besteht darin, daß man den Katalysator im Reaktor vor Einsatz mit HF vorbehandelt, z. B. indem man den Fluorwasserstoff in verdünnter Form mit Methanol anwendet.

Das erfindungsgemäße Verfahren wird vorzugsweise mit Aluminosilikatzeolithen und Borosilikatzeolithen ausgeführt.

Eine bevorzugte Ausführungsform besteht darin, den Zeolithen vor oder nach der Verstrangung mit Bindemittel 1 bis 3 h unter Rückfluß in einer 0,001n bis 1n HF, bevorzugt 0,05 bis 0,5n HF, zu behandeln. Nach Abfiltrieren und Auswaschen wird bei 100 bis 140°C getrocknet und bei 500° bis 600°C calciniert. An diesem vorangehend beschriebenen Katalysator wird Methanol und/oder Dimethylether bei einem Druck zwischen Normaldruck und ca. 30 bar, vorzugsweise bei 0 bis 1 bar und bei Temperaturen zwischen 300° und 600°C, bevorzugt zwischen 350° und 500°C für Aluminosilikatzeolithe oder 400 und 550°C für Borsilikatzeolithe umgesetzt. Das Methanol kann einen Wassergehalt bis zu 90 Gew.-% haben. Dem Methanol können auch noch andere niedere Alkohole beigemischt sein. Die Belastung des Katalysators ausgedrückt in WHSV = $h^{-1}$ — g Methanol und/oder Dimethylether pro g Katalysator und Stunde — wird vorteilhafterweise so gewählt, daß diese Einsatzstoffe möglichst quantitativ umgesetzt werden, damit keine Abtrenn- und Rückführprobleme mit Dimethyläther entstehen. Im allgemeinen wird daher WHSV im Bereich von 0,5 bis 50 $h^{-1}$, vorzugsweise von 1 bis 10 $h^{-1}$ bzw. 2 bis 15 $h^{-1}$ liegen.

Ein besonderer Vorteil des erfindungsgemäßen Verfahrens ist die Erhöhung der Laufzeit der verwendeten Katalysatoren. Unter Laufzeit versteht man die Zeit zwischen zwei Regenerierungen. Auch die Lebensdauer des Katalysators wird insgesamt verlängert. Der erfindungsgemäße Effekt der Laufzeitverbesserung wirkt sich besonaers bei der Umsetzung bei hohen Temperaturen z. B. im Bereich von 450 bis 500°C aus. Die Fluorwasserstoff-Behandlung hat neben dem standzeitverbessernden Einfluß auf den Katalysator noch den Effekt, daß die Bildung der unerwünschten Nebenprodukte Methan und Aromaten stark zurückgedrängt wird. Es ist ein weiterer Vorteil der Erfindung, daß man die Umsetzung von Rohmethanol bzw. Dimethylether in $C_2-C_4$-Olefine ohne Verdünnungsmittel ausführen kann.

Die Durchführung des erfindungsgemäßen Verfahrens wird anhand der nachstehenden Beispiele näher erläutert.

## Beispiel 1

Ein Aluminosilikatzeolith wird in einer hydrothermalen Synthese hergestellt aus 65 g $SiO_2$ (Aerosil 200), 475 g $Al(OH)_3$ in 800 g einer wäßrigen 1,6-Hexandiamin-Lösung (Mischung 50 : 50) bei 150°C unter autogenem Druck in einem Rührautoklaven. Nach Abfiltrieren und Auswaschen wird das kristalline Produkt bei 160°C 24 h getrocknet und bei 500°C 24 h calciniert. Dieser Aluminosilikatzeolith setzt sich zusammen aus 92,6 Gew.-% $SiO_2$ und 5,6 Gew.-% $Al_2O_3$.

Daraus werden zwei Versuchskatalysatoren A und B gewonnen.

Katalysator A wird hergestellt, indem der Aluminiumzeolith mit Boehmit als Bindemittel im Verhältnis 60 : 40 verstrangt, bei 110°C 16 h getrocknet und bei 500°C 20 h calciniert wird.

Katalysator B wird hergestellt, indem 75 g des Katalysators A in 200 ml einer 0,1n HF-Lösung 1 bis 3 h unter Rückfluß behandelt werden. Nach Abfiltrieren und gutem Auswaschen wird das erhaltene Produkt bei 110°C 16 h getrocknet und bei 500°C 20 h calciniert.

Nachfolgend werden die beiden Ausführungsformen des unbehandelten (A) und des HF-behandelten Katalysators an Methanol getestet. In der Tabelle 1 wird das Ergebnis der Umwandlung von 25%igem Methanol bei T = 400°C und WHSV = 1,4 h$^{-1}$ zu niederen Olefinen beschrieben. Das Ende der Laufzeit des Katalysators wird durch den Dimethylether-Durchbruch angezeigt.

Tabelle 1

|  | Katalysator | |
|---|---|---|
|  | A | B |
| $CH_4$ | 1,7% | 1,4% |
| $C_2H_4$ | 18,0% | 15,7% |
| $C_2H_6$ | 0,3% | 0,3% |
| $C_3H_6$ | 22,5% | 19,8% |
| $C_3H_5$ | 4,7% | 6,0% |
| $C_4H_8$ | 15,5% | 13,6% |
| $C_4H_{10}$ | 11,4% | 14,9% |
| $C_5{}^+$ | 24,0% | 26,4% |
| Laufzeit*) | 26 h | 38 h |
| g $CH_3OH$/g Katalysator | 36 g | 53 g |

*) Laufzeit = Zeit zwischen zwei Regenerierungen.

Die Prozentgehalte geben die Ausbeuten bezogen auf eingesetztes $CH_2$ wieder.

Der Vergleich der Beispiele zeigt den standzeitverbessernden Einfluß, den eine Fluorwasserstoff-Behandlung des verwendeten Katalysators bewirkt.

## Beispiel 2

Ein Borosilikatzeolith wird in einer hydrothermalen Synthese hergestellt aus 64 g $SiO_2$ (Aerosil 200), 12,16 g $H_3BO_3$ in 800 g einer wäßrigen 1,6-Hexandiamin-Lösung (Mischung 50 : 50) bei 150°C unter autogenem Druck in einem Rührautoklaven. Nach Abfiltrieren und Auswaschen wird das kristalline Produkt bei 160°C 24 h getrocknet und bei 500°C 24 h calciniert. Dieser Borosilikatzeolith setzt sich zusammen aus 88,1 Gew.-% $SiO_2$ und 3,27 Gew.-% $B_2O_3$. Man stellt daraus wiederum zwei Versuchskatalysatoren A und B her.

Katalysator A wird hergestellt, indem der Borzeolith mit Boehmit als Bindemittel im Verhältnis 60 : 40 verstrangt, bei 110°C 16 h getrocknet und bei 500°C 20 h calciniert wird.

Katalysator B erhält man, indem man 75 g des Borzeolithen in 200 ml einer 0,1n HF-Lösung 1 bis 3 h unter Rückfluß behandelt. Nach Abfiltrieren und gutem Auswaschen wird das erhaltene Produkt bei 110°C 16 h getrocknet und bei 500°C 20 h calciniert. Danach erfolgt eine Verstrangung mit Boehmit als Bindemittel im Verhältnis 60 : 40, ein abermaliges Trocknen (110°C/16 h) und Calcinieren (500°C/20 h).

In der nachfolgenden Tabelle 2 wird die Umwandlung von Rohmethanol an den Katalysatorformen A und B zu niederen Olefinen gemessen. Das Methanol wird bei der Reaktion jeweils quantitativ umgesetzt.

Tabelle 2

| | Katalysator A | B | A | B |
|---|---|---|---|---|
| Temperatur | 450°C | 450°C | 500°C | 500°C |
| WHSV | $7,8 \, h^{-1}$ | $7,8 \, h^{-1}$ | $7,8 \, h^{-1}$ | $7,8 \, h^{-1}$ |
| $CH_4$ | 2,7% | 0,6% | 9,3% | 1,9% |
| $C_2H_4$ | 6,2% | 4,1% | 9,2% | 7,2% |
| $C_2H_6$ | 0,2% | 0,1% | 0,8% | 0,2% |
| $C_3H_6$ | 35,2% | 30,2% | 39,7% | 33,7% |
| $C_3H_8$ | 2,0% | 0,9% | 1,6% | 1,2% |
| $C_4H_8$ | 18,8% | 17,3% | 19,5% | 18,3% |
| $C_4H_{10}$ | 4,1% | 3,8% | 1,8% | 1,5% |
| $C_5{}^+$ | 27,5% | 39,3% | 15,7% | 30,8% |
| Laufzeit*) | 45 h | 79 h | 11 h | 48 h |
| g $CH_3OH$/g Katalysator | 390 g | 616 g | 86 g | 374 g |

*) Laufzeit bedeutet Zeit zwischen zwei Regenerierungen.

Die Prozentgehalte geben die Ausbeuten bezogen auf eingesetztes $CH_2$ wieder.

Die Ergebnisse der Vergleichsversuche belegen den standzeitverbessernden Einfluß, den die erfindungsgemäße Fluorwasserstoff-Behandlung bei dem verwendeten Borosilikat-Katalysator bewirkt. Der positive Effekt kommt besonders bei höheren Temperaturen stark zur Geltung. Auch wird deutlich, daß die Methanbildung gerade bei hoher Temperatur von 500°C beträchtlich zurückgedrängt wird.

**Patentansprüche**

1. Verfahren zur Herstellung von Olefinen durch Umsetzung von Methanol und/oder Dimethylether bei erhöhter Temperatur in Gegenwart von Zeolithkatalysatoren, dadurch gekennzeichnet, daß man mit Fluorwasserstoff behandelte Zeolithkatalysatoren vom Pentasiltyp verwendet.

2. Verfahren nach Anspruch 1, dadurch gekennzeichnet, daß man als Katalysator einen Aluminosilikatzeolithen verwendet.

3. Verfahren nach Anspruch 2, dadurch gekennzeichnet, daß man die Umsetzung von Methanol und/oder Dimethylether bei Temperaturen von 350 bis 500°C und einem Druck zwischen Normaldruck und 30 bar durchführt.

4. Verfahren nach Anspruch 1, dadurch gekennzeichnet, daß man als Katalysator einen Borosilikatzeolithen verwendet.

5. Verfahren nach Anspruch 4, dadurch gekennzeichnet, daß man die Umsetzung von Methanol und/oder Dimethylether bei Temperaturen von 400 bis 500°C und bei einem Druck zwischen Normaldruck und 30 bar durchführt.

6. Verfahren nach den Ansprüchen 1 bis 5, dadurch gekennzeichnet, daß man den Zeolithkatalysator mit 0,001 n bis 1 n Lösung von Fluorwasserstoff behandelt.

7. Verfahren nach Anspruch 6, dadurch gekennzeichnet, daß man den Katalysator im Reaktor vor

**0 072 920**

dem Einsatz mit Fluorwasserstoff vorbehandelt.

8. Verfahren nach Anspruch 7, dadurch gekennzeichnet, daß man die Behandlung mit einer methanolischen Lösung vornimmt.

## Claims

1. A process for the preparation of olefins by converting methanol and/or dimethyl ether at elevated temperature in the presence of a zeolite catalyst, wherein a zeolite catalyst of the pentasil type which has been treated with hydrogen fluoride is used.

2. A process as claimed in claim 1, wherein an aluminosilicate zeolite is used as the catalyst.

3. A process as claimed in claim 2, wherein the reaction of methanol and/or dimethyl ether is carried out at from 350 to 500° C and under a pressure between atmospheric pressure and 30 bars.

4. A process as claimed in claim 1, wherein a borosilicate zeolite is used as the catalyst.

5. A process as claimed in claim 4, wherein the reaction of methanol and/or dimethyl ether is carried out at from 400 to 500° C and under a pressure between atmospheric pressure and 30 bars.

6. A process as claimed in claims 1 to 5, wherein the zeolite catalyst is treated with a 0.001 N—1 N solution of hydrogen fluoride.

7. A process as claimed in claim 6, wherein the catalyst is pre-treated with hydrogen fluoride in the reactor before use.

8. A process as claimed in claim 7, wherein the treatment is carried out with a methanolic solution.

## Revendications

1. Procédé pour la production d'oléfines par transformation de méthanol et/ou d'éther diméthylique à température élevée et en présence de catalyseurs zéolitiques, caractérisé en ce qu'on utilise des catalyseurs zéolitiques du type pentasile, traités par l'acide fluorhydrique.

2. Procédé selon la revendication 1, caractérisé en ce qu'on utilise une zéolite d'aluminosilicate comme catalyseur.

3. Procédé selon la revendication 2, caractérisé en ce qu'on mène la réaction du méthanol et/ou de l'éther diméthylique à des températures de 350 à 500°C et sous une pression comprise entre la pression atmosphérique standardisée et 30 bars.

4. Procédé selon la revendication 1, caractérisé en ce qu'on utilise une zéolite de borosilicate comme catalyseur.

5. Procédé selon la revendication 4, caractérisé en ce qu'on mène la réaction du méthanol et/ou de l'éther diméthylique à des températures de 400 à 500°C et sous une pression comprise entre la pression atmosphérique standardisée et 30 bars.

6. Procédé selon l'une quelconque des revendications 1 à 5, caractérisé en ce qu'on traite le catalyseur zéolitique par une solution 0,001 n à 1 n d'acide fluorhydrique.

7. Procédé selon la revendication 6, caractérisé en ce qu'on traite le catalyseur préalablement par l'acide chlorhydrique dans le réacteur avant le chargement de celui-ci.

8. Procédé selon la revendication 7, caractérisé en ce qu'on procède au traitement avec une solution méthanolique.

5